# EUROPÄISCHE PATENTANMELDUNG

(11) **EP 1 312 365 A1**
(43) Veröffentlichungstag der Anmeldung: **21.05.2003**
(21) Anmeldenummer: 02002882.5
(22) Anmeldetag: 08.02.2002
(51) Int. Cl.: A61K 31/519, A61K 45/06, A61P 15/10

(54) **Pteridinone zur Behandlung der erektilen Dysfunktion**

(30) Priorität: 20.11.2001 US 989948
(71) Anmelder: Jenapharm GmbH & Co. KG, 07745 Jena (DE)
(72) Erfinder: Schröder, Jens, Dr., 07743 Jena (DE); Saad, Farid, Dr., 13359 Berlin (DE)
(74) Vertreter: Störle, Christian, Dr.

(57) **Zusammenfassung**

Die Anmeldung beschreibt die Verwendung von Pteridinonen der Formel (1) 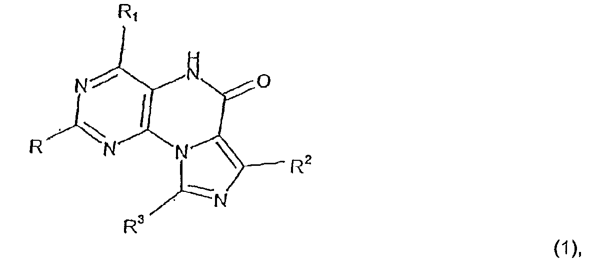 in der
R für 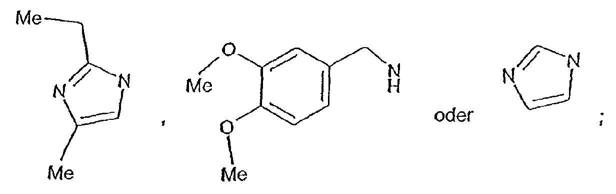
R¹ für Wasserstoff oder ein C₁-C₄-Alkyl, insbesondere Ethyl oder iso-Propyl;
R² für Wasserstoff oder C₁-C₄ -Alkyl, insbesondere Methyl, und
R³ für C₁-C₄-Alkyl, insbesondere Ethyl oder Cyclohexylethyl
stehen, und/oder von pharmazeutisch verträglichen Salzen dieser Pteridinone zur Herstellung eines Arzneimittels zur Behandlung der erektilen Dysfunktion.

## Beschreibung

Die Erfindung bezieht sich auf die Verwendung von speziellen Pteridinonen und deren pharmazeutisch verträglichen Salzen zur Behandlung der erektilen Dysfunktion.

Die erektile Dysfunktion (Impotenz) ist weltweit ein großes medizinisches Problem erwachsener Männer im reproduktiven und sexuell aktiven Alter. Impotenz kann definiert werden als die Unfähigkeit des Mannes zur Kopulation bzw. zur Ejakulation oder beidem zusammen. Die *Massachusetts Male Aging Study* hat folgende Verteilung von moderaten und schweren erektilen Funktionsstörungen festgestellt: 22% der 40-jährigen, 32% der 50-jährigen, 40% der 60-jährigen und 49% der 70-jährigen Männer sind erkrankt. Schätzungen gehen weltweit von 100 bis 200 Millionen Betroffenen aus. Mit der steigenden Lebenserwartung wird auch diese Zahl in den nächsten Jahren weiter anwachsen.

Die erektile Reaktion wird nach psychischen und/oder physischen Stimuli durch parasympathische Impulse ausgelöst (endogene NO-Freisetzung), welche letztendlich die Spiegel der cyclischen Nukleotide cAMP und cGMP im Penis erhöhen und dadurch eine Dilatation der Penisarterien und eine Erschlaffung der Muskulatur des Corpus Cavernosum bewirken. Die Akkumulation von cAMP und cGMP ist ein Schlüsselschritt der penilen Erektion. Sie werden aus GTP und ATP unter Einwirkung der Enzyme Guanylat- und Adenylatcyclase produziert. Auf der anderen Seite bauen Phosphodiesterasen (PDE) diese cyklischen Nukleotide ab.

Aufgrund der vorstehend geschilderten enormen medizinischen Bedeutung der erektilen Dysfunktion wurden natürlich verschiedene Wirkstoffe entwickelt, mit denen diese Krankheit behandelt werden kann Dabei wurde die Forschung und Entwicklung auf spezifische PDE-Inhibitoren fokussiert, also auf einen Inhibitor, der spezifisch eine der sechs Phosphodiesterasen PDE I, PDE II, PDE III, PDE IV, PDE V und PDE VI hemmt. Ein Beispiel dafür ist der in EP 0 702 555 B1 beschriebene Stoff Sildenafil, der unter der Bezeichnung Viagra® als Arzneimittel vermarktet wird. Sildenafil hemmt spezifisch PDE V. Allerdings ist bei Verwendung spezifischer PDE-Inhibitoren, z.B. Sildenafil, die Wirkung nicht stark genug und die Wirkungsdauer ist nicht ausreichend lang.

Der vorliegenden Erfindung liegt deshalb die Aufgabe zugrunde, die Symptome der erektilen Dysfunktion ohne die vorstehenden Nachteile behandeln zu können.

Erfindungsgemäß wird dies erreicht durch Verwendung von Pteridinonen der Formel (1) 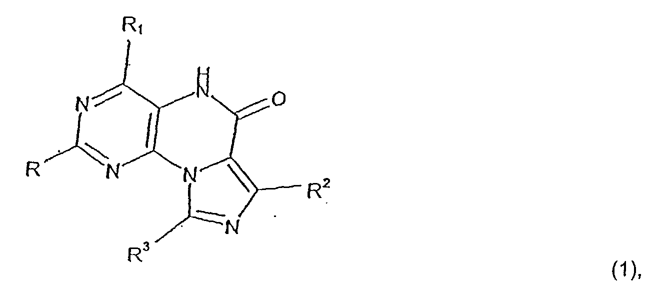 in der R für 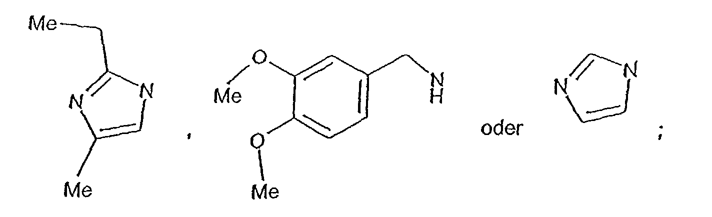
R¹ für Wasserstoff oder ein C₁-C₄-Alkyl, insbesondere Ethyl oder iso-Propyl;
R² für Wasserstoff oder C₁-C₄-Alkyl, insbesondere Methyl, und
R³ für C₁-C₄-Alkyl, insbesondere Ethyl oder Cyclohexylethyl
stehen, und/oder von pharmazeutisch verträglichen Salzen dieser Pteridinone zur Herstellung eines Arzneimittels zur Behandlung der erektilen Dysfunktion.
Bei den Substituenten R erfolgt die Bindung über die freie Valenz am N-Atom.

Wie bereits vorstehend ausgeführt wurde, kann die erektile Dysfunktion definiert werden als die Unfähigkeit des Mannes zur Kopulation bzw. zur Ejakulation oder beidem zusammen. Letztendlich ist bei der erektilen Dysfunktion entweder gar keine oder nur eine verminderte Erektion des Penis festzustellen.

Die vorliegende Erfindung beruht nun auf der vollkommen überraschenden Erkenntnis, daß die erektile Dysfunktion besonders gut mit den vorstehend beschriebenen Pteridinonen und/oder deren pharmazeutisch verträglichen Salzen behandelt werden kann. Dabei wurde vorteilhafterweise eine sehr ausgeprägte Wirkung beobachtet, wobei die Wirkung über einen ausgesprochen langen Zeitraum anhielt.

Bei Pteridinonen, wie sie erfindungsgemäß verwendet werden, handelt es sich um an sich bekannte Verbindungen, wie sie beispielsweise in der EP 0 429 149 A1 beschrieben sind. Dort ist auch detailliert die Herstellung von Pteridinonen erläutert. In der EP 0 429 149 A1 wird auf die cardiovasculären Eigenschaften der darin beschriebenen Pteridinone abgestellt, es findet sich in ihr aber kein Hinweis, daß Pteridinone zur Behandlung der erektilen Dysfunktion eingesetzt werden können.

Es wurde nun überraschenderweise gefunden, daß es sich bei den erfindungsgemäß eingesetzten Pteridinonen um nicht selektive Phosphodiesterase-Inhibitoren handelt, d.h. um mischfunktionelle PDE-Inhibitoren, die mehr als einen der bekannten PDE-Subtypen inhibieren. Günstigerweise werden zumindest die PDE-Subtypen IV und V gehemmt, zusätzlich können auch noch andere PDE-Subtypen, insbesondere PDE I und/oder PDE VI inhibiert werden. So wurde beispielsweise festgestellt, daß die Verbindung 9-Ethyl-2-(2-ethyl-4-methyl-1H-imidazol-1-yl)-7-methyl-4-(2-propyl)imidazo[5,1-h]pteridin-6(5H)-on die PDE-Typen I, IV, V und VI hemmt.

Es wird angenommen, ohne an diese Annahme gebunden zu sein, daß durch die nicht selektive Hemmung verschiedener Phosphodiesterase-Typen eine Akkumulation von sowohl cAMP als auch cGMP erreicht wird. Durch Konzentrationserhöhung dieser beiden cyclischen Nukleotide wird eine im Gegensatz zu spezifischen Phosphodiesterase-Inhibitoren, z.B. Sildenafil, deutlich stärkere Wirkung und eine wesentlich längere Wirkungsdauer erreicht. Da im Vergleich zu spezifischen Inhibitoren die Wirkungsdauer länger und die Wirkung stärker ist, können zur erfolgreichen Behandlung der erektilen Dysfunktion geringere Dosierungen verwendet werden, wodurch es zu weniger Nebenwirkungen kommt, da Nebenwirkungen im allgemeinen bei einer höheren Dosierung beobachtet werden.

Durch diese unspezifische Hemmung verschiedener Phosphodiesterase-Subtypen sind die vorstehend angegebenen Pteridinone besonders gut zur Behandlung der erektilen Dysfunktion geeignet.

Durch die beschriebene starke Wirkung und die erreichbare lange Wirkungsdauer kann insbesondere bei Patienten mit hohem Risikopotential, wie Diabetes, Bluthochdruck und Multiple Sklerose, eine vorbeugende Dauerbehandlung mit dem vorgenannten Pteridinon in niedriger Dosierung zur Aufrechterhaltung der erektilen Dysfunktion durchgeführt werden.

In einer bevorzugten Ausführungsform der erfindungsgemäßen Verwendung handelt es sich bei dem erfindungsgemäß eingesetzten Pteridinon um 9-Ethyl-2-(2-ethyl-4-methyl-1H-imidazol-1-yl)-7-methyl-4-(2-propyl)imidazo[5,1-h]pteridin-6(5H)-on, 2-[[3,4-Dimethoxyphenyl)methyl]amino]-4,9-diethyl-7-methylimidazo[5,1-H]pteridin-6(5H)-on, 9-Ethyl-2-(2-ethyl-4-methyl-1H-imidazol-1-yl)-7-methylimidazo[5,1-H]pteridin-6(5H)on oder 9-(2-Cyclohexylethyl)-4-ethyl-2-(1H-imidazol-1-yl)imidazo[5,1-H]pteridin-6(5H)-on. Diese Verbindungen zeigen überraschenderweise eine besonders starke Wirkung, die außerdem auch sehr lange andauert.

Erfindungsgemäß werden unter Verwendung der vorstehenden Pteridinone Arzneimittel hergestellt. Die Arzneimittel können dabei 1 Pteridinon oder mehrere, voneinander verschiedene Pteridinone enthalten.

Zur Herstellung der Arzneimittel können pharmazeutisch verträgliche Salze der vorstehenden Pteridinone eingesetzt werden. Solche Salze sind insbesondere Phosphate, Sulfate, Citrate, Chloride und Bromide sowie Hydrochloride (Anlagerung von HCI an Pteridinone). Das erfindungsgemäß hergestellte Arzneimittel kann 1 Salz oder mehrere voneinander verschiedenen Salze der Pteridinone aufweisen. Das Arzneimittel kann 1 Pteridinon oder mehrere Pteridinone als freie Basen und/oder ein oder mehrere Pteridinone als pharmazeutisch verträgliche Salze enthalten.

Vorzugsweise liegt im Arzneimittel weiterhin ein NO-Donor vor. Dabei werden unter dem Ausdruck "NO-Donor" Verbindungen jeglicher Art verstanden, die im Körper NO freisetzen können. Beispiele solcher NO-Donoren sind S-Nitroso-N-acetylpenicillamin oder Nitroprussidnatrium. Das Arzneimittel kann mehrere, voneinander verschiedene NO-Donoren aufweisen.

Das Arzneimittel kann dabei so ausgestaltet sein, daß es die Verabreichung des NO-Donors vor, zusammen mit oder nach Applikation der Pteridinone ermöglicht. Soll der NO-Donor beispielsweise zusammen mit dem Pteridinon dem Patienten gegeben werden, können diese beiden Wirkstoffe in einer pharmazeutischen Formulierung, beispielsweise einer Tablette, gemeinsam vorliegen. Dabei werden NO-Donor und Pteridinon günstigerweise so gewählt, daß sie nicht miteinander reagieren. Soll der NO-Donor vor oder nach Applikation der Pteridinone dem Patienten verabreicht werden, so kann das Arzneimittel in Form eines Kits ausgestaltet sein, wobei ein Teil des Kits pharmazeutische Formulierungen enthält, die nur das Pteridinon aufweisen, und ein anderer Teil des Kits solche Formulierungen hat, die nur den NO-Donor umfassen.

Günstigerweise ist das Arzneimittel zur oralen oder parenteralen Verabreichung geeignet, einschließlich topischen, rektalen, subcutanen, intravenösen, intramuskulären, intraperitonealen, intranasalen, intrabukkalen oder sublingualen Applikation. Die erfindungsgemäß hergestellten Arzneimittel können neben den vorstehend beschriebenen Wirkstoffen noch übliche Träger- und Verdünnungsmittel aufweisen. Die Arzneimittel können mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt werden. Die Dosis kann 0,05 mg bis 100 mg pro pharmazeutische Formulierung betragen.

Als pharmazeutische Formulierungen können insbesondere zur Anwendung kommen (die Mengenangaben beziehen sich auf die pharmazeutische Formulierung):
- Tabletten oder Dragees von 0,1 bis 100 mg oral,
- Ampullen von 0,1 bis 100 mg als subkutane Injektion,
- Pflaster mit transdermaler Freisetzung von 0,05 bis 10 mg,
- subkutane Implantate mit Freisetzungskapazität von 0,05 bis 10 mg,
- Gele und Cremen mit transdermaler Freisetzung von 0,05 bis 10 mg,
- bukkal applizierbare Systeme mit einer Freisetzung von 0,1 bis 10 mg.

Besonders bevorzugt ist ein oral verabreichbares Arzneimittel, beispielsweise Tabletten oder Dragees, da die Applikation in besonders einfacher Weise erfolgen kann.

Die Verabreichung kann bei Bedarf, z.B. täglich, erfolgen.

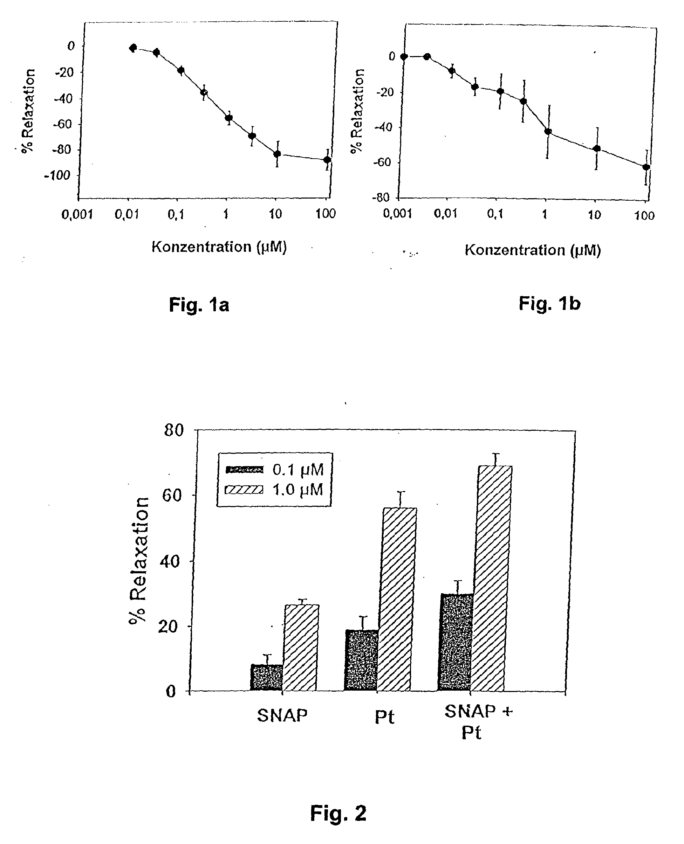

Die vorliegende Erfindung wird nachfolgend anhand von Beispielen, die jedoch nicht einschränkend ausgelegt werden sollen, unter Bezugnahme auf die Figuren näher erläutert, wobei
- Fig. 1:: den Effekt des Pteridinons 9-Ethyl-2-(2-ethyl-4-methyl-1H-imidazol-1-yl)-7-methyl-4-(2-propyl)imidazo[5,1-h]pteridin-6(5H)on auf die Phenylephrien-induzierte Kontraktion in der cavernosalen glatten Muskulatur von Kanninchen (Fig. 1a) sowie von impotenten Patienten (Fig. 1 b) zeigt;
- Fig. 2:: die Relaxation des glatten Muskels des Cavernosums von Kanninchen darstellt, die durch S-Nitroso-N-Acetylpenicillamin (SNAP) und dem Pteridinon 9-Ethyl-2-(2-ethyl-4-methyl-1H-imidazol-1-yl)-7-methyl-4-(2-propyl)imidazo[5,1-h]pteridin-6(5H)on (Pt) induziert wird (die Daten stellen Mittelwerte ± S.E. von 6 Experimenten für SNAP alleine, 12 Experimenten für Pt alleine und 3 Experimenten für eine Kombination von SNAP mit Pt dar);

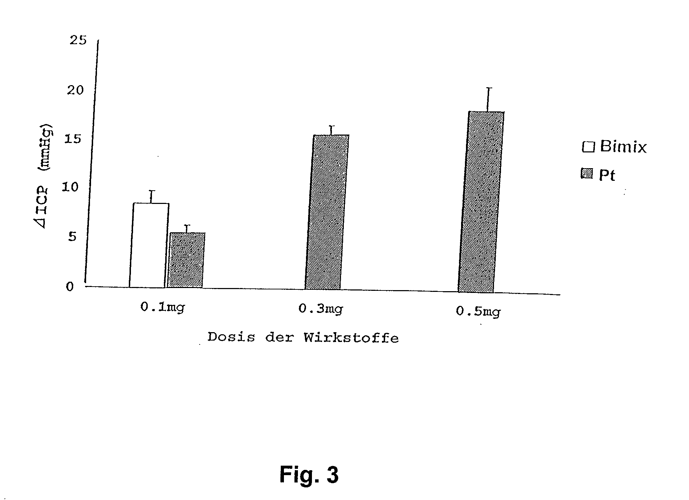

- Fig. 3:: eine vergleichende Studie unter Verwendung von Bimix (Papaverin und Phentolamin) und dem Pteridinon 9-Ethyl-2-(2-ethyl-4-methyl-1 H-imidazol-1-yl)-7-methyl-4-(2-propyl)imidazo[5,1-h]pteridin-6(5H)on (Pt) auf den intracavernosalen Druck in Katzen nach lokaler intra-arterieller Injektion zeigt (Daten stellen Mittelwerte ± S.E. dar (n=7 für jede Gruppe mit 0,1 und 0,3 mg/Tier, n=6 bei 0,5 mg/Tier); die Körpergewichte der Tiere der experimentellen Gruppen waren ähnlich);

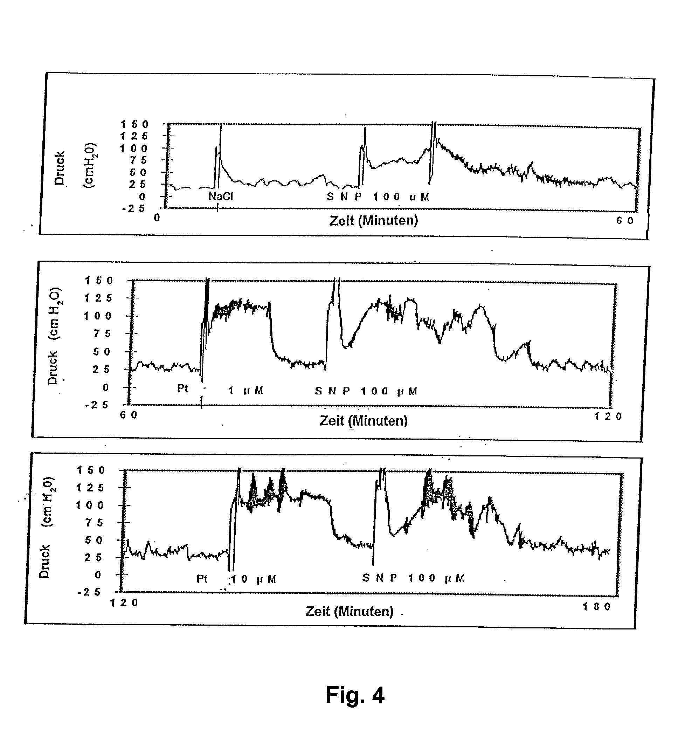

- Fig. 4:: eine Aufzeichnung des intracavernosalen Blutdrucks von Natriumnitroprussid (SNP; 0,5 ml, 10⁻⁴ M) und dem Pteridinon 9-Ethyl-2-(2-ethyl-4-methyl-1H-imidazol-1-yl)-7-methyl-4-(2-propyl)imidazo[5,1-h]pteridin-6(5H)on (Pt) (0,5 ml, 10⁻⁶ M oder 0,5 ml 10⁻⁵ M) in Affen darstellt, und

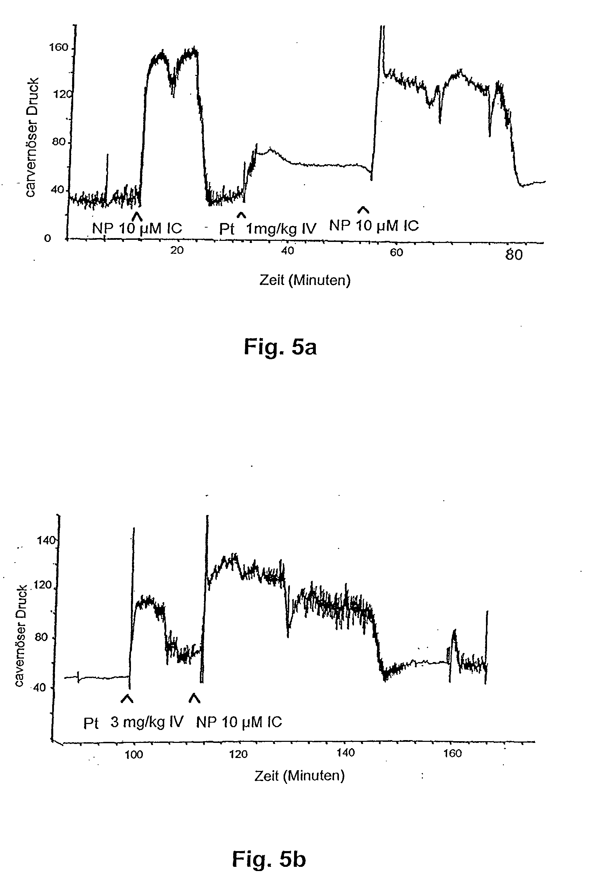

- Fig. 5:: den Effekt von 9-Ethyl-2-(2-ethyl-4-methyl-1 H-imidazol-1-yl)-7-methyl-4-(2-propyl)imidazo[5,1-h]pteridin-6(5H)on (Pt) nach i.v. Verabreichung an Affen zeigt, (die Verabreichung des Pteridinons bei Dosen von 1 und 3 mg/kg i.v. erhöhte den intracavernosalen Druck (in cm H₂O); die Vorbehandlung mit dem Pteridinon verlängerte die Dauer der Nitroprussid(NP)-Antwort (10⁻⁵ M i.c.)).

In den nachfolgenden Beispielen wird, soweit nichts anderes angegeben ist, ausschließlich das Pteridinon 9-Ethyl-2-(2-ethyl-4-methyl-1H-imidazol-1-yl)-7-methyl-4-(2-propyl)imidazo[5,1-h]pteridin-6(5H)on verwendet, das der Einfachheit halber nur als "tricyclisches Pteridinon" bezeichnet wird.

### Beispiel 1: Bestimmung des Phosphodiesterase-Subtyp-Profils

Die verschiedenen Formen cyclischer Nukleotidphosphodiesterasen (PDE) wurden mit üblichen Methoden isoliert. Die PDE-lsozyme wurden aus den folgenden Quellen isoliert: PDE I und PDE V aus Rinder-Aorta, PDE II aus dem linken Ventrikel von Ratten, PDE IV aus Rattenniere und PDE III aus menschlichen Plättchen.

Die PDE-Aktivität wurde in einem Reaktionsmedium gemessen, das 40 mM Tris-HCI (pH 8,0), 5 mM MgCl₂, 1 mM Dithiothreitol und 0,2 µM [³H] cyclisches GMP enthielt. Das tricyclische Pteridinon wurde in DMSO (2,5%) gelöst. Bei dieser DMSO-Konzentration wurde die Enzymaktivität zu etwa 10% inhibiert. Die IC₅₀-Werte für das tricyclische Pteridinon wurden anhand von Dosis-Wirkungs-Kurven bestimmt, bei denen die Konzentration im Bereich von 1 nM bis 10 µM (ganze log-Inkremente) lag. Für jedes untersuchte PDE-Isozym wurden mindestens zwei IC₅₀-Bestimmungen an verschiedenen Tagen durchgeführt.

Das tricyclische Pteridinon zeigt eine inhibitorische Aktivität gegenüber den PDE-Typen I, IV, V und VI. Die Inhibierung von PDE I und V erhöht cGMP, während die Inhibierung von PDE IV cAMP erhöht.

**Tabelle 1:**

| PDE-Subtyp-Profil des tricyclischen Pteridinons (IC50 in nM) | | | | | | |
|---|---|---|---|---|---|---|
| | **PDE I** | **PDE II** | **PDE III** | **PDE IV** | **PDE V** | **PDE VI** |
| tricyclisches Pteridinon | 5,8 | 5.600 | >10.000 | 74 | 35 | 56 |

Es ist bekannt, daß das erektile Gewebe beim Menschen die PDE-Typen II, III, IV und V enthält. Daher ist zu erwarten, daß das tricyclische Pteridinon aufgrund der PDE IV- und V-Inhibierung sowohl cGMP als auch cAMP in der cavernösen, glatten Muskulatur des Menschen erhöht. Interessanterweise verbessert die spezifische PDE I-Inhibierung (Vinpocetin) die kognitive Funktion.

### Beispiel 2: Kontraktionsstudien mit cavernösem Kaninchen- bzw. Humangewebe

Männliche New Zealand White-Kaninchen (Gewicht: 2,5-3,0 kg) wurden mit CO₂-Gas eingeschläfert. Das cavernöse Gewebe wurde beim Sezieren in eine HEPES-gepufferte Lösung (20°C, pH 7,4) eingebracht. Die oben liegende Skelettmuskulatur wurde vorsichtig entfernt, um nicht die darunterliegende Tunica albuginea zu beschädigen. Im proximalen Ende der Tunica wurde ein Schnitt ausgeführt, während sie distal erweitert wurde. Dann wurde das Corpus Cavernosum bilateral aus der Tunica herausgelöst.

Für menschliches Gewebe wurde das Verfahren zum Ernten von Gewebe vom Institutional Review Board for Human Studies am Boston University Medical Center genehmigt. Cavernöses Gewebe wurde von Patienten erhalten, die sich der Implantation einer Penisprothese zur Behandlung einer erektilen Dysfunktion unterzogen. Im Operationssaal wurden Biopsien der Corpora cavernosa sofort in eisgekühlte (4°C) physiologische Salzlösung gelegt und zum Labor transportiert. Gewebestreifen mit einer Größe von etwa 3 mm x 3mm x 10 mm wurden zugeschnitten und für Organbadstudien gemäß einem ähnlichen Verfahren wie dem nachfolgend beschriebenen präpariert.

Längsschnitte des Corpus cavernosum wurden in Organbäder gelegt, die folgendes (in mM) enthielten: 118 NaCI, 4,7 KCI, 1,2 KH₂PO₄, MgSO₄, 24,9 NaCO₃, 5,5 Glucose, 2,0 Na-Pyruvat und 2,0 CaCl₂. Die Streifen cavernöser, glatter Muskulatur wurden mit 2 Gramm gestreckt und mit einem Gemisch von 95% O₂ und 5% CO₂ während zwei Stunden äquilibriert, bevor mit dem Versuchsverfahren begonnen wurde. Dann wurden die Gewebe an Kraftwandler (Grass FT03) angeschlossen. Die Spannungsmessungen wurden mit einem MP100-System und der AcqKnowledge-Software (Biopac) digital aufgezeichnet.

Die Gewebe wurden mit Indomethacin (10 µM) vorbehandelt, um Cyclooxygenaseeffekte zu beseitigen. Durch Zugabe von Phenylephrin (10 µM) zu den Organkammern wurden Kontraktionen induziert. Nachdem die Phenylephrin-induzierte Kontraktion einen dauerhaften Zustand erreicht hatte, wurde das tricyclische Pteridinon (Pt) (Fig. 1a und 1b) oder der Stickstoffoxid-Donor S-Nitroso-N-acetylpenicillamin (SNAP) zugegeben (Fig. 2).

Bei dem in Fig. 1 beschriebenen Experiment wurde festgestellt, daß das tricyclische Pteridinon die Phenylephrin-induzierte (10⁻⁵) Kontraktion in konzentrationsabhängiger Weise wirksam entspannt. Der IC₅₀-Wert für die Inhibierung der Phenylephrin-induzierten Kontraktion durch das tricyclische Pteridinon betrug ≈ 1 µM. Indomethacin (10⁻⁵ M) war bei allen Experimenten vorhanden. Bei den Untersuchungen an den Kaninchen stellen die Daten eine mittlere ±-Standardabweichung von 7-13 Experimenten dar (Ausnahme: nur bei 2 Experimenten wurde das tricyclische Pteridinon bei 100 µM getestet; die Werte sind hinsichtlich der Zeitkontrolle korrigiert). Bei den Untersuchungen mit humanem Gewebe wurde das tricyclische Pteridinon zweimal unter Verwendung von Gewebe impotenter Männer getestet.

Sowohl das tricyclische Pteridinon als auch der Stickstoffoxid-Donor SNAP bewirkten eine unmittelbare Relaxation der kavernösen, glatten Muskulatur bei Kaninchen (Fig. 2). Die sofortige Relaxation legt nahe, daß die basale Produktion von cAMP und cGMP in erektilem Gewebe ausreicht, um eine rasche pharmakologische Reaktion hervorzurufen.

Im Hinblick auf therapeutische Überlegungen hat die Kombination von tricyclischem Pteridinon und SNAP einen additiven Effekt (Fig. 2). Somit legen die Ergebnisse nahe, daß das tricyclische Pteridinon nicht nur unabhängig von Guanylatcyclase-Agonisten arbeiten, sondern auch die Stickstoffoxid-vermittelte Relaxation verstärken kann.

Es wurde festgestellt, daß das tricyclische Pteridinon bei diesem *in vitro*-Modell der erektilen Funktion stärker war als der Stickstoffoxid-Donor SNAP (Fig. 2). In Kombination mit SNAP hat das tricyclische Pteridinon einen additiven Effekt.

### Beispiel 3: Katzenmodell der männlichen erektilen Dysfunktion

Geschlechtsreife männliche Katzen (Körpergewicht 2,9-4,6 kg) wurden mit α-Chloralose (60 mg/kg i.m.) narkotisiert. Dann wurden Hartmansche Lösung und eine Dextroselösung bei 2 µl/kg/h über die antekubitale Vene infundiert. Die Beatmung wurde über eine an einen Tierventilator (Harvard Apparatus Ltd., Boston MA) angeschlossene Tracheotomie sichergestellt. Pancuroniumbromid (0,3 ml/kg) wurde als Muskelrelaxans infundiert. Nach Bedarf wurden zusätzliche Dosen von α-Chlorase und Pancuroniumbromid gegeben, um ein gleichbleibendes Anästhesie-Niveau beizubehalten.

In der inneren Carotid-Arterie wurde ein zentraler Katheter gelegt, um eine Arterienblut-Gasanalyse (ABGA) durchzuführen und den systemischen Blutdruck in der Arterie aufzuzeichnen. Eine 23 G-Nadel wurde in das Corpus cavernosum eingeführt, um die Veränderungen des intracavernösen Drucks (ICP) zu messen. Sowohl der systemische, arterielle Blutdruck als auch der ICP wurden mittels eines Druckwandlers (Gilson P23XL-1, physiologischer Druckwandler, Ohmeda, Kanada), der an einen Polygraphen (Grass Model 7D, Quincy, MA, USA) angeschlossen war, gemessen. Zur lokalen Injektion von Versuchsverbindungen wurde ein Zweig der inneren Pudendalarterie auf einer Seite mit einem länglichen Polyethylenschlauch (PE-10-Schlauch, Original-Innendurchmesser 0,25 mm, Außendurchmesser 0,76 mm) retrograd mikrokanüliert. Dann wurden zur Verabreichung der Wirkstoffe (jeweils 0,1 ml) in den Penis die übrigen Zweige, bis auf die Penisarterie, ligiert. Mit geeigneten Ventilator-Einstellungen (z.B. Tidalvolumen 10 ml/kg; Atmungsrate 15-20/Min.) wurde eine Normoxie (pH > 7,25, PO₂ > 60 mmHg, PCO₂ < 40 mmHg) erzeugt und mit ABGA und einem mit der ausgeatmeten Luft verbundenen Capnometer (Modell 2200, Traverse Medical Monitors, USA) überprüft. Um die Volumeneffekte von Lösungsmitteln zu beurteilen, wurden destilliertes Wasser und normale Kochsalzlösung intraarteriell infundiert. Intraarterielles Wasser und Kochsalzlösung hatten selbst bei einem Bolus keinen Effekt auf den intracavernösen Druck (ICP).

Die Lösungen des tricyclischen Pteridinon wurden unter Verwendung einer 1%igen 6N HCI- und einer 5%igen DMSO-Lösung hergestellt. Papaverin (50 mg) + Phentolamin (5 mg) (Bimix) wurde in 5 ml mit 5% DMSO/Rest Kochsalzlösung hergestellt. Jedes Arzneimittel wurde als Bolus von 0,1 ml und 0,05 ml heparinisierter Kochsalzlösung zum Spülen infundiert.

Der Grund-ICP, ΔICP (d.h. die reine Veränderung des Grund-ICP) und Veränderungen des systemischen Blutdrucks wurden nach der intraarteriellen Injektion aufgezeichnet. Alle Daten wurden mittels eines Student's t-Test beurteilt, wobei p<0,05 als signifikant galt. Die Daten priapischer Episoden wurden bei den Berechnungen ausgeschlossen.

Bei einer anfänglichen Dosisermittlungsstudie wurde festgestellt, daß das tricyclische Pteridinon den ICP nach lokalen intraarteriellen Injektionen von 0,0038 mg (0,1 ml, Bolus) oder mehr erhöht.

Eine lokale intraarterielle Verabreichung des tricyclischen Pteridinon (0,1 mg/Tier) erzeugte einen signifikanten Anstieg des ICP von 5,5 mmHg (p<0,01). Bei 0,1 mg/Tier wurden die durch das tricyclische Pteridinon bewirkten Erektionen nicht als steif (visuelle Bestimmung) erachtet. Die einzige Dosis der Bimix-Lösung, die getestet wurde, betrug 0,1 mg/Tier. Höhere Dosen von Bimix erforderten ein zu großes Volumen, das zur Solubilisierung des Injektats nötig war, so daß keine weiteren Versuche durchgeführt wurden. Es ist auch zu beachten, daß das Bimix nicht oral verfügbar ist. Nach i.v. Bolus-Injektion (0,5 mg/Tier) induzierte das tricyclische Pteridinon vorübergehende (≤ 5 Minuten) Spitzenabfälle des systemischen Arterienblutdrucks von 15,8 mmHg.

Das tricyclische Pteridinon erhöhte in dosisabhängiger Weise signifikant den ICP (Fig. 3). Zusätzlich wurde festgestellt, daß das tricyclische Pteridinon bei diesen höheren Dosen zu einer steifen Erektion führte. Die Ergebnisse zeigen deutlich, daß das tricyclische Pteridinon bei diesem Katzenmodell der erektilen Funktion ein wirksames Arzneimittel ist. Die Dauer der erektilen Reaktion war deutlich länger als der vorübergehende Effekt auf den Blutdruck, was nahelegt, daß die Wirksamkeit bei zirkulierenden Plasmaniveaus auftreten kann, die nominale hämodynamische Effekte haben.

### Beispiel 4: Affenmodell der männlichen erektilen Dysfunktion

Das tricyclische Pteridinon wurde *Macaca fascicularis*-Affen als intracavernöse Bolusinjektion (0,5 ml, mit 0,5 ml Kochsalzlösung-Flush) verabreicht. Die Auswirkungen auf den intracavernösen Druck wurden mitlaufend gemessen und mit denen des Stickstoffoxid-Donors Nitroprussid (SNP oder NP) verglichen. Stickstoffoxid induziert eine Erektion, indem es als Guanylatcyclase-Agonist wirkt.

Wenn das tricyclische Pteridinon vor der Verabreichung von Nitroprussid verabreicht wurde, verursachte es keinen anhaltenden Anstieg des cavernösen Drucks bei Affen. Dies läßt möglicherweise darauf schließen, daß die Aktivierung der Adenylat- und Guanylatcyclase-Aktivität durch Neurotransmitter, die von parasympathischen und nicht-adrenergen, nichtcholinergen (NANC) Nerven freigesetzt werden (wie dies bei psychogenen oder physischen Stimuli der Fall wäre), für die Verstärkung der erektilen Reaktion bei diesem Modell durch das tricyclische Pteridinon notwendig ist. Wenn das tricyclische Pteridinon kurz nach der Rückkehr der Nitroprussid-induzierten Erhöhung des intracavernösen Drucks auf die Grundwerte verabreicht wurde, bewirkte es eine dosisabhängige Erhöhung des maximalen und des mittleren intracavernösen Drucks (Tabelle 2, Fig. 4).

**TABELLE 2:**

| Auswirkung des tricyclischen Pteridinon auf den intrakavernösen Druck bei Affen | | | |
|---|---|---|---|
| | Tricyclisches Pteridinon | | |
| [Molar] | 10⁻⁶ | 10⁻⁵ | 10⁻⁴ |
| Maximaler Druck [cm H₂O] | 95 (2) | 103 (3) | 165 (1) |
| Mittlerer Druckanstieg [Δ cm H₂O] | 41 (2) | 51 (3) | 62 (1) |

Die Zahlen in Klammern geben die Anzahl der Versuchsaffen an.

In einer zweiten Versuchsreihe wurde festgestellt, daß eine Co-Injektion von tricyclischem Pteridinon und Nitroprussid wirksamer war als eine aufeinanderfolgende Verabreichung der Verbindungen. Zusätzlich verlängerte die gleichzeitige Injektion die Dauer des Nitroprussid-induzierten Anstiegs des cavernösen Drucks in dosisabhängiger Weise. Eine gleichzeitig verabreichte Dosis des tricyclischen Pteridinon bei 1 mg/kg verdoppelte die Dauer, während 3 mg/kg die Dauer nahezu verdreifachten. Bei einem Affen führte eine Vorbehandlung mit dem tricyclischen Pteridinon in einer Dosis von 3 mg/kg, gefolgt von Nitroprussid (10⁻⁵ M i.c. Injektion) zu einem Priapismus, der erfolgreich mit Phenylephrin (75 µg i.c. Injektion) behandelt wurde.

Die abschließende Reihe der *in vivo*-Versuche an Affen untersuchte die Effekte des tricyclischen Pteridinon bei intravenöser Verabreichung (1 und 3 mg/kg) 15 Minuten vor der intracavernösen Injektion von Nitroprussid (10⁻⁵ M). Diese Vorgehensweise wurde gewählt, um zu bestimmen, ob die "systemische Verabreichung" von tricyclischem Pteridinon die Nitroprussid-induzierte, erektile Reaktion wirksam verstärkt.

Durch die systemische Verabreichung von tricyclischem Pteridinon wurde der intracavernöse Druck beim Affen wirksam erhöht (Fig. 5). Die Erhöhung des intracavernösen Drucks nach der systemischen Verabreichung von tricyclischem Pteridinon zeigt, daß ein Anstieg des intracavernösen Drucks abhängig von der Zeit, welche die Verbindung benötigt, um nach oraler Verabreichung in denn Blutstrom zu gelangen, auftritt. Pharmakokinetische Versuche zeigen, daß signifikante Plasmamengen 30 Minuten oder weniger nach oraler Verabreichung zu beobachten sind, was darauf schließen läßt, daß die Verbindung einen raschen therapeutischen Effekt hat.

Nach i.v. Verabreichung in den hohen Dosen von 1 und 3 mg/kg wurde eine Blutdrucksenkung von 20-40 mmHg beobachtet.

## Patentansprüche

1. Verwendung von Pteridinonen der Formel (1) 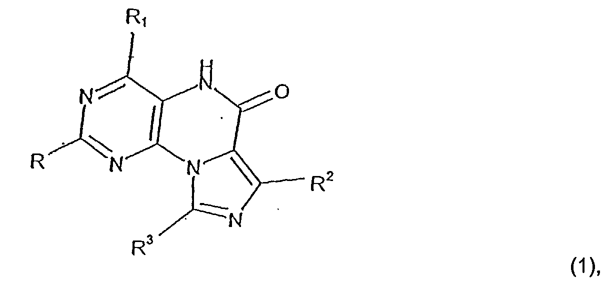 in der R für 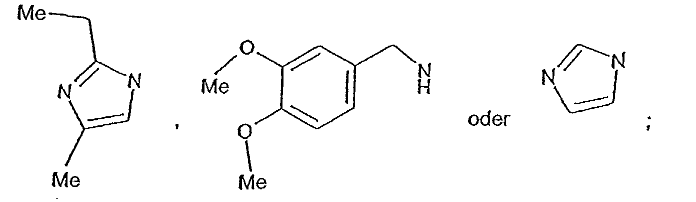
R¹ für Wasserstoff oder ein C₁-C₄-Alkyl, insbesondere Ethyl oder iso-Propyl;
R² für Wasserstoff oder C₁-C₄-Alkyl, insbesondere Methyl, und
R³ für C₁-C₄-Alkyl, insbesondere Ethyl oder Cyclohexylethyl
stehen, und/oder von pharmazeutisch verträglichen Salzen dieser Pteridinone zur Herstellung eines Arzneimittels zur Behandlung der erektilen Dysfunktion.

2. Verwendung nach Anspruch 1, wobei das Pteridinon 9-Ethyl-2-(2-ethyl-4-methyl-1H-imidazol-1-yl)-7-methyl-4-(2-propyl)imidazo[5,1-h]pteridin-6(5H)on, 2-[[3,4-Dimethoxyphenyl)methyl]amino]-4,9-diethyl-7-methylimidazo[5,1-H]pteridin-6(5H)-on, 9-Ethyl-2-(2-ethyl-4-methyl-1H-imidazol-1-yl)-7-methylimidazo[5,1-H]pteridin-6(5H)on, 9-(2-Cyclohexylethyl)-4-ethyl-2-(1H-imidazol-1-yl)imidaza[5,1-H]pteridin-6(5H)-on ist.

3. Verwendung nach einem der vorhergehenden Ansprüche, wobei im Arzneimittel weiterhin ein NO-Donor vorliegt.

4. Verwendung nach einem der vorhergehenden Ansprüche, wobei das Arzneimittel zur oralen oder parenteralen Verabreichung geeignet ist.
